# EUROPEAN PATENT SPECIFICATION

(11) **EP 2 633 079 B1**
(45) Date of publication and mention of the grant of the patent: **12.08.2015**
(21) Application number: 11799810.4
(22) Date of filing: 19.10.2011
(51) Int. Cl.: C12Q 1/68

(54) **METHOD AND KIT FOR THE DIAGNOSIS OF IGA NEPHROPATHY**
VERFAHREN UND KIT ZUR DIAGNOSE VON IGA-NEPHROPATHIE
MÉTHODE ET NÉCESSAIRE DE DIAGNOSTIC DE LA NÉPHROPATHIE À IGA

(30) Priority: 28.10.2010 IT MI20102007
(43) Date of publication of application: 04.09.2013
(73) Proprietor: Università degli Studi di Bari "Aldo Moro", 70121 Bari (BA) (IT)
(72) Inventor: SALLUSTIO, Fabio, 70124 Bari (BA) (IT); SERINO, Grazia, 70124 Bari (BA) (IT); SCHENA, Francesco Paolo, 70124 Bari (BA) (IT)
(74) Representative: Trupiano, Federica
(86) International application number: PCT/IB2011/002494
(87) International publication number: WO 2012/056282

(56) References cited:
- EP-A1- 1 085 090
- EP-A1- 2 133 431
- WO-A2-2009/130479
- US-A1- 2006 019 286
- WANG GANG ET AL: "Expression of microRNAs in the urinary sediment of patients with IgA nephropathy", DISEASE MARKERS, vol. 28, no. 2, 2 April 2010 (2010-04-02), pages 79-86, XP008136141, online ISSN: 0278-0240
- WANG GANG ET AL: "Intrarenal expression of microRNAs in patients with IgA nephropathy", LABORATORY INVESTIGATION, vol. 90, no. 1, 9 November 2009 (2009-11-09), pages 98-103, XP002635188, online ISSN: 0023-6837
- XIE LIN-SHEN ET AL: "The role of C1GALT1C1 in lipopolysaccharide-induced IgA1 aberrant O-glycosylation in IgA nephropathy", CLINICAL AND INVESTIGATIVE MEDICINE, vol. 33, no. 1, February 2010 (2010-02), pages E5-E13, XP008136143, ISSN: 0147-958X

### SEQUENCE LISTING

<110> UNIVERSITA DEGLI STUDI DI BARI
<120> Metodo e kit per la diagnosi della IgA nefropatia
<130> 11564
<150> MI2010A002007
   <151> 2010-10-28
<160> 18
<170> PatentIn version 3.5
<210> 1
   <211> 22
   <212> RNA
   <213> Homo sapiens
<220>
   <221> misc_RNA
   <222> (1)..(22)
   <223> Mature sequence of human micro RNA precursor miR-148b
<400> 1
   ucagugcauc acagaacuuu gu 22
<210> 2
   <211> 22
   <212> RNA
   <213> Homo sapiens
<220>
   <221> misc_RNA
   <222> (1)..(22)
   <223> mature sequence of human micro RNA precursor let-7b
<400> 2
   ugagguagua gguugugugg uu 22
<210> 3
   <211> 22
   <212> RNA
   <213> Homo sapiens
<220>
   <221> misc_RNA
   <222> (1)..(22)
   <223> mature sequence of human micro RNA precursor let-7d
<400> 3
   agagguagua gguugcauag uu 22
<210> 4
   <211> 23
   <212> RNA
   <213> Homo sapiens
<220>
   <221> misc_RNA
   <222> (1)..(23)
   <223> mature sequence of human micro RNA precursor miR-361
<400> 4
   ucccccaggu gugauucuga uuu 23
<210> 5
   <211> 21
   <212> RNA
   <213> Homo sapiens
<220>
   <221> misc_RNA
   <222> (1)..(21)
   <223> mature sequence of human micro RNA precursor miR-886
<400> 5
   cgcgggugcu uacugacccu u 21
<210> 6
   <211> 21
   <212> RNA
   <213> Homo sapiens
<220>
   <221> misc_RNA
   <222> (1)..(21)
   <223> mature sequence of human micro RNA precursor mir-188
<400> 6
   caucccuugc augguggagg g 21
<210> 7
   <211> 22
   <212> DNA
   <213> Homo sapiens
<220>
   <221> misc_RNA
   <222> (1)..(22)
   <223> Complementary antisense RNA sequence of miRNA miR-148b (long sequence)
<400> 7
   acaaagttct gtgatgcact ga 22
<210> 8
   <211> 19
   <212> DNA
   <213> Homo sapiens
<220>
   <221> misc_RNA
   <222> (1)..(19)
   <223> complementary antisense RNA sequence of miRNA miR-148b (short sequence)
<400> 8
   acaaagttct gtgatgcac 19
<210> 9
   <211> 22
   <212> DNA
   <213> Homo sapiens
<220>
   <221> misc_RNA
   <222> (1)..(22)
   <223> Complementary antisense RNA sequence of miRNA miR-let-7b (long sequence)
<400> 9
   aaccacacaa cctactacct ca 22
<210> 10
   <211> 19
   <212> DNA
   <213> Homo sapiens
<220>
   <221> misc_RNA
   <222> (1)..(19)
   <223> complementary antisense RNA sequence of miRNA miR-let-7b (short sequence)
<400> 10
   aaccacacaa cctactacc 19
<210> 11
   <211> 22
   <212> DNA
   <213> Homo sapiens
<220>
   <221> misc_RNA
   <222> (1)..(22)
   <223> Complementary antisense RNA sequence of miRNA miR-let-7d (long sequence)
<400> 11
   aactatgcaa cctactacct ct 22
<210> 12
   <211> 19
   <212> DNA
   <213> Homo sapiens
<220>
   <221> misc_RNA
   <222> (1)..(19)
   <223> complementary antisense RNA sequence of miRNA miR-let-7d (short sequence)
<400> 12
   aactatgcaa cctactacc 19
<210> 13
   <211> 23
   <212> DNA
   <213> Homo sapiens
<220>
   <221> misc_RNA
   <222> (1)..(23)
   <223> Complementary antisense RNA sequence of miRNA hsa-mir-361 (long sequence)
<400> 13
   aaatcagaat cacacctggg gga 23
<210> 14
   <211> 19
   <212> DNA
   <213> Homo sapiens
<220>
   <221> misc_RNA
   <222> (1)..(19)
   <223> Complementary antisense RNA sequence of miRNA hsa-mir-361 (short sequence)
<400> 14
   aaatcagaat cacacctgg 19
<210> 15
   <211> 21
   <212> DNA
   <213> Homo sapiens
<220>
   <221> misc_RNA
   <222> (1)..(21)
   <223> Complementary antisense RNA sequence of miRNA hsa-mir-886 (long sequence)
<400> 15
   aagggtcagt aagcacccgc g 21
<210> 16
   <211> 19
   <212> DNA
   <213> Homo sapiens
<220>
   <221> misc_RNA
   <222> (1)..(19)
   <223> Complementary antisense RNA sequence of miRNA hsa-mir-886 (short sequence)
<400> 16
   aagggtcagt aagcacccg 19
<210> 17
   <211> 21
   <212> DNA
   <213> Homo sapiens
<220>
   <221> misc_RNA
   <222> (1)..(21)
   <223> Complementary antisense RNA sequence of miRNA hsa-mir-188 (long sequence)
<400> 17
   ccctccacca tgcaagggat g 21
<210> 18
   <211> 19
   <212> DNA
   <213> Homo sapiens
<220>
   <221> misc_RNA
   <222> (1)..(19)
   <223> Complementary antisense RNA sequence of miRNA hsa-mir-188 (short sequence)
<400> 18
   ctccaccatg caagggatg 19

## Description

### Summary of the invention

The present invention concerns a new method for the diagnosis of glomerulonephritis with mesangial deposits of IgA (IgA nephropathy) based on the detection of an increase in the expression levels of some microRNAs (mi-RNAs). The invention also concerns use of the mi-RNAs for the diagnosis of the disease, a kit for diagnostic use and use of the antisense mi-RNAs for the treatment and/or prevention of the disease.

### Technical background

IgA nephropathy, the most common primitive renal disease, occurs at any age, but in particular in children and young adults. It is six times more frequent in males than in females. Its prevalence, compared with that of all the other primitive glomerulopathies, is 5% in the USA, 10-20% in Southern Europe and in Australia and 30-40% in Asia. In approximately 50% of patients with recurrent haematuria, mesangial deposits of IgA are found and in approximately half of these patients there is an increase in serous IgA. Alterations in the lymphocytic subpopulations have been documented. The data suggest that IgA nephropathy is caused by increased production and/or reduced clearance of the IgA-antigene polymeric complexes which deposit in the mesangium of the glomeruli. The polymeric IgA probably derive from the surfaces of the mucous membranes and from the bone marrow.

The pathogenetic mechanism of this disease is still unknown, however genetic factors appear to play a key role in susceptibility to the disease. Mesangial deposits of deglycosylated IgAl at glomerular level and recurrent episodes of macroscopic haematuria in conjunction with infections of the upper respiratory tract or persistent microhaematuria with or without proteinuria constitute the elements for diagnosis. Up to now, IgA nephropathy can be diagnosed for certain only by renal biopsy, an invasive procedure and therefore not easily applicable as a routine analysis for diagnostic purposes. In patients or family members of patients with persistent microhaematuria only, it is not easy to perform renal biopsy as it is often refused by the subject.

Therefore, there is the need for new biological markers which can improve the diagnostic possibilities with the identification of diseases in the early stage without performing renal biopsy.

In recent years, in medical research particular interest has been aroused by small molecules of RNA called microRNA (mi-RNA) which regulate the expression of genes, inhibiting the transcription of messenger RNA in proteins or favouring degradation of the messenger RNA of specific genes. It has been shown that a single mi-RNA expression profile exists for every disease and that the altered expression of one or more mi-RNA is specific to each pathology. It has been suggested for example that gene expression profiles, in particular microRNA profiling, are useful in both the diagnosis and prognosis of cancer.

Wang Gang et al., Disease Markers, vol 28, n. 2, 2 April 2010 pages 79-86, disclose the detection of miRNA levels in urine samples of IgA nephopathy subjects and suggest that the expression of miRNAs can be considered as non-invasive marker of kidney disease.

Wang Gang et al., Laboratory Investigation, vol 90, n. 1, 9 November 2009 pages 98-103, disclose the intrarenal expression of miRNAs in nephropathy subjects and concluded that miRNA species may be important in the pathogenesis and progression of IgA nephropathy.

EP 1085090 discloses the use of antisense oligonucleotides to inhibit DNA transcription or mRNA translation coding for proteins having activity related to IgA nephropathy for the treatment of the disease.

Xie Lin-Shen et al., Clinical and Investigative Medicine, vol. 33, n. 1, February 2010, pages E5-E13, disclose the functional role of c1GALT1 in aberrant IgAA1 o-glycosilation induced by lipopolysaccharide and identify potential therapeutic targets in IgA nephropathy.

### Objects of the invention

The object of the present invention is to provide a diagnostic method for IgA nephropathy which is not invasive, which can therefore be used in a routine manner and which is readily accepted by the patient, thus overcoming the drawbacks of the prior art methods.

A further aim of the invention is to provide a kit for performing the method of the invention.

A further aim of the invention is to provide a medicament for the treatment and prevention of IgA nephropathy.

### Description of the invention

These and further aims are achieved by the invention which, according to one of its embodiments, concerns a mi-RNA which has a sequence chosen from the sequences SEQ. ID No.: 1-6 for use in the diagnosis of glomerulonephritis with mesangial deposits of IgA, below also referred to simply as "IgA nephropathy".

The mi-RNAs used according to the invention are chosen from the following:
- hsa-miR-148b : UCAGUGCAUCACAGAACUUUGU (SEQ. ID No. 1)
- hsa-let-7b : UGAGGUAGUAGGUUGUGUGGUU (SEQ. ID No. 2)
- hsa-let7d : AGAGGUAGUAGGUUGCAUAGUU (SEQ. ID No. 3)
- hsa-miR-361-3p : UCCCCCAGGUGUGAUUCUGAUUU (SEQ. ID No. 4)
- hsa-miR-886-3p : CGCGGGUGCUUACUGACCCUU (SEQ. ID No. 5)
- hsa-miR-188-5p : CAUCCCUUGCAUGGUGGAGGG (SEQ. ID No. 6) According to another of its embodiments, the invention concerns a mi-RNA which has a sequence chosen from the sequences SEQ. ID No.: 1-6 for use as a marker of IgA nephropathy.

The terms "diagnosis" and "diagnose" here indicate verification both of the evident IgA disease and the predisposition to develop the disease.

According to a preferred embodiment of the invention, the mi-RNA is miR-148b having the sequence SEQ. ID No 1.

According to another of its embodiments, the invention concerns a method for the diagnosis of IgA nephropathy which comprises evaluating the expression of at least one mi-RNA chosen from the sequences SEQ. ID No.: 1-6 in a biological sample of a subject, in which an increase in the expression of said mi-RNA indicates the presence of IgA nephropathy or a predisposition to develop IgA nephropathy. According to a preferred embodiment of the invention, the method is characterized by the fact that said at least one mi-RNA is miR-148b having the sequence SEQ. ID No. 1.

According to a particularly preferred embodiment of the invention, the method is characterized by the fact that it comprises evaluation of the expression of miR-148b having the sequence SEQ. ID No. 1 and at least one other mi-RNA chosen from the sequences SEQ. ID No. 2-6, or the expression of miR-148b and at least another two, three, four or five mi-RNAs of the sequences SEQ. ID No. 2-6 or alternatively all six mi-RNAs as defined above.

The term "expression" as used here refers to the quantity of a particular sequence of mi-RNA which is transcribed by its locus, in other words the concentration of the mi-RNA in the sample analysed.

According to the present invention, it is considered that there is an "increase in the expression" of mi-RNA if the levels of expression of said mi-RNA in the sample, with respect to a control sample, are higher by at least 5%, preferably 10%, preferably at least 20-30% and more preferably 35-50%.

The term "biological sample" here indicates an element isolated from the body of the subject on which the method of the invention can be applied, i.e. a biological sample containing the mi-RNA defined above. Said biological sample is preferably whole blood, for example peripheral blood, the peripheral lymphomonocytes, the serum, the plasma, or samples taken from the oral mucosa.

According to the invention the biological sample is chosen from whole blood, for example peripheral blood, the peripheral lymphomonocytes, the serum and the plasma, advantageously from peripheral blood, peripheral lymphomonocytes and serum, for example from the peripheral lymphomonocytes and the serum. An experimental evaluation was performed on the expression of the miR-148b in the serum and in the lymphomonocytes which were found to be consistent; in fact, in both cases the serum levels of the miR-148b were significantly higher than those of the healthy subjects, as shown in the accompanying figures and in the comments on them, in the experimental part of the present description. It is evident, furthermore, that the use of the serum for evaluation of the mi-RNA as markers for IgA nephropathy at diagnostic level would be particularly practical and economically affordable, as it avoids the phase of isolation of the peripheral lymphomonocytes from the blood.

In the method of the invention, the level of expression of each mi-RNA can be determined by normalizing its expression level with respect to an internal control in the same subject so as to obtain a normalized expression value called ΔCt. This will represent a predictive type threshold value.

According to another of its embodiments, the invention concerns a diagnostic kit for use in the diagnosis of IgA nephropathy which comprises at least one antisense mi-RNA with respect to the miRNAs of the SEQ. ID No.: 1-6.

According to the present invention, two molecules of nucleic acid (i.e. the "sense" molecule and the "antisense" molecule) can be perfectly complementary, meaning that they do not contain any mismatched base and/or additional nucleotides. According to this embodiment of the invention, therefore, the antisense mi-RNAs are composed of the sequences SEQ.ID.No. 7-18, perfectly complementary with respect to the mi-RNA sequences having the sequences SEQ.ID.No. 1-6. Alternatively, the antisense molecules can have some mismatched bases (i.e. some bases that do not pair with the corresponding bases of the sense mi-RNA) or can differ in the number of total nucleotides (due to additions or deletions).

According to a preferred embodiment of the invention, the antisense molecules are perfectly complementary with the molecules of mi-RNAs having the sequences SEQ.ID.No. 1-6.

In particular, the antisense mi-RNAs for the use according to the invention have a sequence chosen from the following sequences SEQ. ID No.: 7-18:
- anti-miR-148b: ACAAAGTTCTGTGATGCACTGA (SEQ. ID No. 7), ACAAAGTTCTGTGATGCAC (SEQ. ID No. 8)
- anti-let-7b: AACCACACAACCTACTACCTCA (SEQ. ID No. 9), AACCACACAACCTACTACC (SEQ. ID No. 10)
- anti-let-7d: AACTATGCAACCTACTACCTCT (SEQ. ID No. 11), AACTATGCAACCTACTACC SEQ. ID No. 12)
- anti-miR-361-3p: AAATCAGAATCACACCTGGGGGA (SEQ. ID No. 13), AAATCAGAATCACACCTGG (SEQ. ID No. 14)
- anti-miR-886-3p: AAGGGTCAGTAAGCACCCGCG (SEQ. ID No. 15), AAGGGTCAGTAAGCACCCG (SEQ. ID No. 16)
- anti-miR-188-5p: CCCTCCACCATGCAAGGGATG (SEQ. ID No. 17), CTCCACCATGCAAGGGATG (SEQ. ID No. 18).

According to a preferred embodiment of the invention, the kit of the invention is characterized by the fact that said at least one antisense mi-RNA is miR-148b-antisense chosen from the sequences SEQ. ID No 7 and SEQ. ID No. 8.

According to a particularly preferred embodiment of the invention, the diagnostic kit according to the invention is characterized by the fact that it comprises miR-148b-antisense having the sequence SEQ. ID No 7 or SEQ. ID No. 8 and at least one other antisense mi-RNA chosen from the sequences SEQ. ID No. 9-18, or that it comprises sense miR-148b and at least another two or more antisense mi-RNAs of the sequences SEQ. ID No. 9-18 or alternatively all twelve antisense mi-RNAs as defined above.

The antisense mi-RNA for the kit according to the invention can be variously marked, for example with fluorescent molecules. The marked antisense mi-RNA for the kit according to the invention constitute a further subject of the present invention. The kit of the invention designed to detect the mi-RNA can for example use Real-Time PCR and can therefore comprise, in addition to a plate for Real-Time, the primers (probes) marked with fluorescent molecules which specifically determine the expression of each mi-RNA, the reagents for the extraction of the mi-RNA, the reagents for the reverse transcription and those necessary for the amplification reaction, as is well known to the person skilled in the art.

The term "primer" or "probe" here refers to the sequences of anti-sense mi-RNAs as defined above.

According to one embodiment, the kit of the invention for detection of the mi-RNAs having the sequences SEQ. ID No.: 1-6 can for example use a hybridization assay in a solution based on acquisition in chemiluminescence. The kit can comprise a pool of probes, preferably antisense mi-RNAs chosen from the sequences SEQ. ID No. 7-18, a plate coated in primary antibodies (Ab) (for example a multi-well plate for ELISA test) able to recognize and bind double strand sequences, secondary antibodies marked with an enzyme able to recognize the occurred primary Ab double strand sequence link, the reagents necessary for recognition of the primary Ab double strand sequence and secondary Ab complex primary Ab double strand sequence, as is well known to the person skilled in the art. In particular the enzyme used is able to transform colourless soluble substrates into stained products that precipitate in the same point in which the recognition occurred (for example alkaline phosphatase or peroxidase can be used). The occurred reaction can be detected and quantified by means of a luminometer.

According to another of its embodiments, the invention concerns an antisense mi-RNA which has a sequence chosen from the sequences SEQ. ID No.: 7-18 for use in the treatment and/or prevention of IgA nephropathy.

According to an advantageous embodiment of the invention, said antisense mi-RNA is characterized by the fact that it is antisense miR-148b having a sequence chosen from SEQ. ID No 7 and SEQ. ID No. 8 with or without chemical modifications which allow in vivo administration (for example with phosphorothioate or by transformation into LNA (Locked Nucleic Acid)).

According to a particularly preferred embodiment of the invention, for the treatment and/or prevention of IgA nephropathy an antisense miR-148b is used, advantageously chosen from the sequences SEQ. ID No 7 and SEQ. ID No 8 and at least one other antisense mi-RNA chosen from the sequences SEQ. ID No. 9-18, or which comprises sense miR-148b and at least another two, three or more antisense mi-RNAs of the sequences SEQ. ID No. 7-18 or alternatively all the antisense mi-RNAs as defined above.

For use in the treatment according to the invention, the molecules of antisense mi-RNAs as defined above are formulated in pharmaceutical compositions prepared according to the techniques well known to the person skilled in the art administered by means of intravenous infusion.

The targets of the mi-RNA highlighted by the bioinformatic analysis were studied using various databases.

In this way it was found that the mi-RNAs defined above (hsa-miR-148b, hsa-let-7b, hsa-let-7d, hsa-miR-361-3p, hsa-miR-886-3p, hsa-miR-188-5p) are involved in the pathogenesis of IgA nephropathy. Of these, the miR-148b presents as a computational target the core enzyme 1,β-1,3-Galactosyltransferase (c1GALT1), known to be involved in IgA nephropathy, as it is responsible for the altered glycosylation of the IgAl.

The results of expression of the mi-RNA obtained with the microarrays on an independent group of subjects by means of Real-Time PCR performed on mi-RNA extracted from the peripheral lymphomonocytes from the blood of 20 subjects (10 patients with IgA nephropathy and 10 healthy subjects) were then validated. The results of the Real-Time PCR confirmed the results of the microarrays. Also in this case the mi-RNAs in question were expressed significantly and to a greater extent in the subjects with IgA nephropathy than in the healthy subjects.

In the same way, it was evaluated whether the expression of the 6 mi-RNAs as defined above was specific to IgA nephropathy or common to other renal pathologies. For this purpose, the expression levels of the 6 mi-RNAs in the peripheral lymphomonocytes isolated from the whole blood of 5 patients with focal segmental glomerulosclerosis (FSGS) were evaluated via Real-Time PCR, comparing them with the expression values obtained from the patients with IgA nephropathy and from the normal subjects. The results obtained show that the increased expression of the 6 mi-RNAs is specific to IgA nephropathy.

The target genes regulated by the mi-RNAs which had been previously identified by the bioinformatic analysis were then experimentally validated. To do this, we increased or reduced the levels of mi-RNAs in the lymphocytes in culture, derived from controls and patients respectively, via transient transfection with synthetic mi-RNA.

Transfection of the mi-RNAs into the lymphocytes in culture was performed using a cationic liposome-based reagent. Cationic liposomes are artificial lipidic vesicles with positive electric charge, able to incorporate the mi-RNA with negative charge and transport it inside the cells by fusion with the cell membranes or by receptor-mediated endocytosis. The mi-RNA, enclosed in the liposome, is subsequently released into the cytoplasm where it increases (mimic) or reduces (inhibitor) the levels of pre-existing mi-RNA.

To biologically validate the actual involvement of the miR-148b in IgA nephropathy, the levels of miR-148b in the lymphomonocytes in culture, deriving from the whole blood of healthy subjects, were increased by transient transfection using synthetic mi-RNA called mimic. The mimic mi-RNAs are synthetic mi-RNAs designed for insertion in the mi-RNA pathways at the level of mature mi-RNAs. The subsequent analyses of the gene c1GALT1, via Real-Time PCR and Western-Blot, highlighted the actual involvement of the miR-148b in regulation of the c1GALT1. In fact, the expression levels at both transcriptional and proteic level were significantly reduced in the transfected lymphomonocytes.

Surprisingly, the levels of the gene c1GALT1 in the lymphomonocytes isolated from normal subjects after transfection were comparable with the basal levels of the gene in the patients with IgA nephropathy.

To further validate the results obtained from the transfection with mimic mi-RNAs, we transfected the lymphomonocytes isolated from patients with IgA nephropathy with molecules of antisense mi-RNA, having the function of "inhibitors" which silence the endogenous cell mi-RNA. These antisense mi-RNAs are complementary to the mi-RNA sequence and once they have entered the cell they determine an effective functional alteration of the complementary mi-RNA. In the study conducted, the levels of the gene c1GALT1, after transfection, were significantly increased and similar to the basal levels of the normal subjects.

The results of the studies conducted are described below with reference to the accompanying figures.

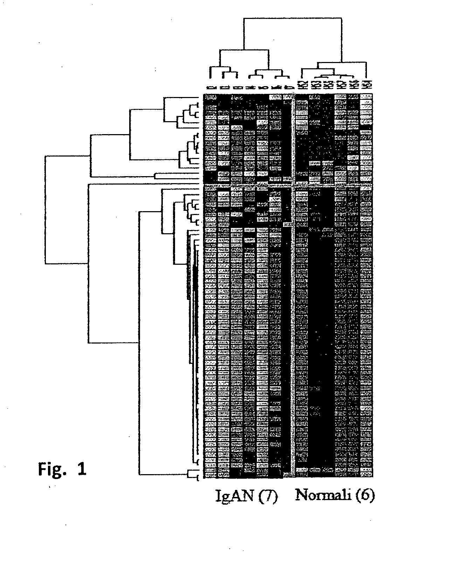

Fig.1 shows the hierarchical clustering of the mi-RNAs deregulated in the 2 classes analysed: patients with IgA nephropathy (7) and healthy subjects (6). From this analysis the clear distinction can be noted between 2 main groups of mi-RNAs which perfectly differentiate the 2 classes analysed (patients with IgA nephropathy and healthy subjects).

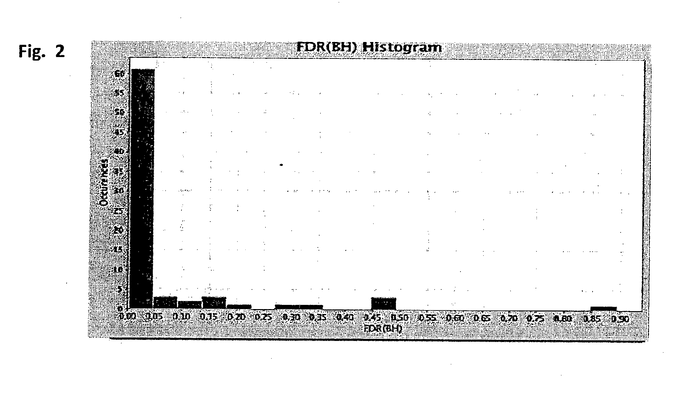

Fig.2 shows the False Discovery Rate (FDR) values of the mi-RNAs found in the expression profile; this analysis was performed to determine both the mi-RNAs that correlate to the greatest extent with IgA nephropathy and the significance of this correlation.

The genes are usually chosen on the basis of a maximum FDR value of 0.05, which indicates that the gene identified has a 5% possibility of being a false positive.

The mi-RNAs with an FDR value below 0.01 were evaluated and only on these were further analyses conducted and further filters introduced, like calculation of the fold change which provides a quantitative value of the expression variation.

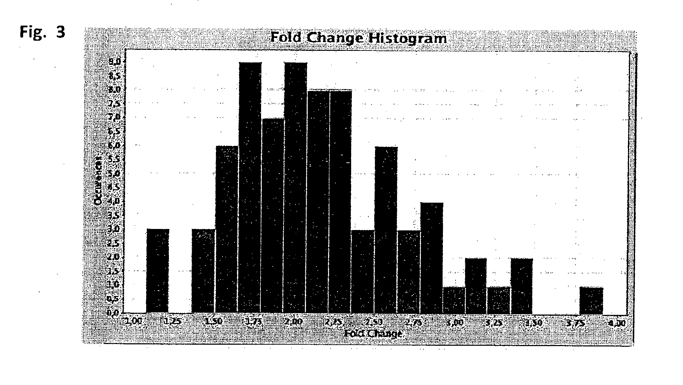

Fig.3 shows the fold change values of the mi-RNAs differently expressed in patients with IgA nephropathy and healthy subjects with an FDR value greater than 0.01. A fold change filter of 2 was applied. Applying both the parameters (FDR and fold change), the 35 mi-RNAs most expressed in the subjects with IgA nephropathy were selected. At this point, the computational targets of each of these mi-RNAs were evaluated by means of bioinformatic programs currently available such as miRanda (http://microrna.sanger.ac.uk/sequences/index.shtml), TargetScan (http://www.targetscan.org), Argonaute (http://www.ma.uni-heidelberg.de), PicTar (http://pictar.bio.nyu.edu) and DianamicroT 3.0 (http://diana.cslab.ece.ntua.gr/microT/).

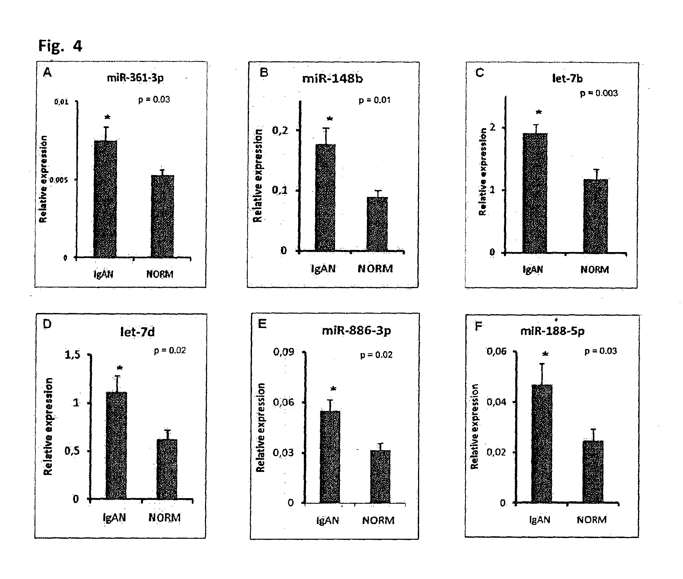

The target genes regulated by the mi-RNAs were analysed by means of the databases Gene Ontology (GO) (www.geneontology.org), KEGG (www.kegg.com) and the software Ingenuity (IPA, Ingenuity System; www.ingenuity.com). The analysis highlighted that 6 mi-RNAs (hsa-miR-148b, hsa-let-7b, hsa-let-7d, hsa-miR-361-3p, hsa-miR-886-3p, hsa-miR-188-5p) are involved in the pathogenesis of IgA nephropathy since their targets were genes that could be involved in the onset and/or progression of the disease. One of these is miR-148b whose computational target is the core enzyme 1,β-1,3-Galactosyltransferase (c1GALT1), known to be involved in IgA nephropathy since it is responsible for the altered glycosylation of the IgA1. Fig.4 shows the expression values of the 6 mi-RNAs (hsa-miR-361-3p, hsa-miR-148b, hsa-let-7b, hsa-let-7d, hsa-miR-886-3p, hsa-miR-188-5p) determined by means of Real-Time PCR on an independent group of 10 patients with IgA nephropathy (IgAN) and 10 healthy subjects (NORM). Each expression value was normalized to the expression value of the miR-27a (a mi-RNA also expressed in the IgAN and NORM samples).

The results are shown as averages of the expression values of each sample ± SEM. As shown in the figure, the expression of the mi-RNAs is significantly higher in the patients with IgA nephropathy compared to the healthy subjects.

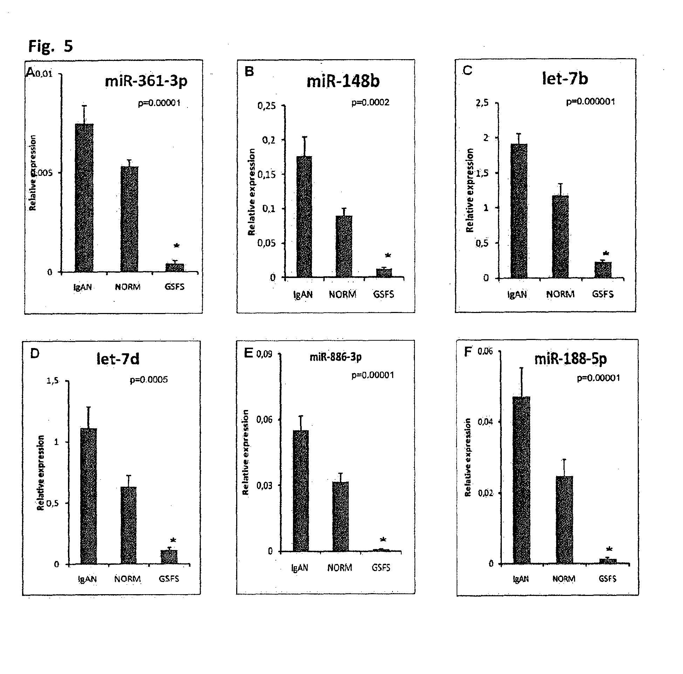

Fig.5 shows the expression values of the 6 mi-RNAs (hsa-miR-361-3p, hsa-miR-148b, hsa-let-7b, hsa-let-7d, hsa-miR-886-3p, hsa-miR-188-5p) determined by means of Real-Time PCR on an independent group of 10 patients with IgA nephropathy (IgAN), 10 healthy subjects (NORM) and 5 patients with focal segmental glomerulosclerosis (FSGS). Each expression value was normalized to the expression value of the miR-27a (a mi-RNA also expressed in the IgAN and NORM samples) and U6 (for the patients with FSGS).

The results are shown as means of the expression values of each sample ± SEM. As shown in the figure, the overexpression of the mi-RNAs is specific to the IgA nephropathy, in fact the expression levels of the 6 mi-RNAs in the patients with focal segmental glomerulosclerosis is significantly reduced with respect to the patients with IgA nephropathy.

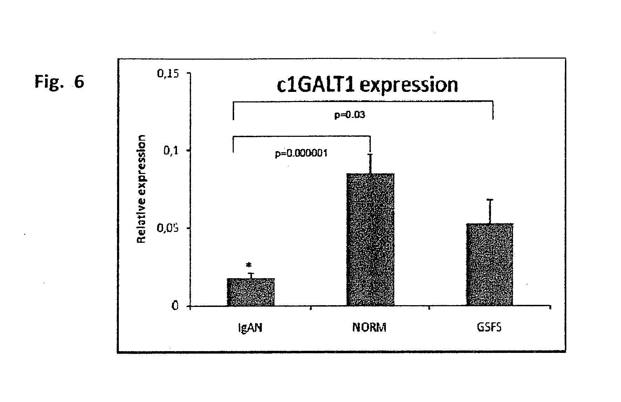

Fig. 6 shows the expression values of the core gene 1,β-1,3-Galactosyltransferase (c1GALT1) in 10 patients with IgA nephropathy, 10 healthy subjects and 5 patients with focal segmental glomerulosclerosis (FSGS), the same as those in which the expression of the 6 mi-RNAs was evaluated. Each expression value was normalized with the expression value of the housekeeping gene β-actin.

The results are shown as means of the expression values of each sample ± SEM.

As shown in the figure, the expression levels of the gene c1GALT1 in the patients with IgA nephropathy are significantly reduced with respect to the normal subjects and patients with FSGS. This reduction, according to the prediction of the in silico analysis, could be due to the increase in the expression of the miR-148b.

To validate the actual involvement of the miR-148b in regulation of the expression of the gene c1GALT1, both the peripheral lymphomonocytes isolated from patients with IgA nephropathy and those of healthy subjects were transfected with molecules of "inhibitor" and "mimic" mi-RNA respectively and subsequently the levels of the gene c1GALT1 were evaluated by means of Real-Time PCR and Western blot.

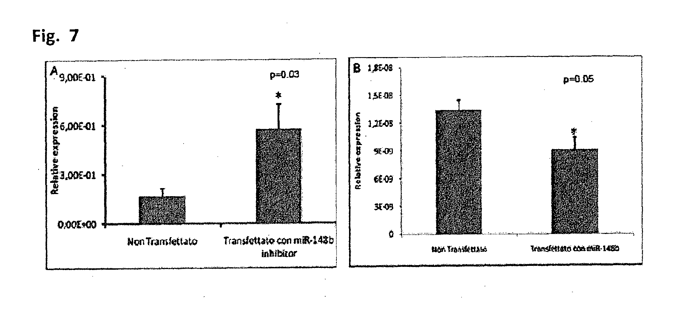

Fig. 7 shows the expression values of the core gene 1,β-1,3-Galactosyltransferase (c1GALT1) in the peripheral lymphomonocytes isolated from 3 patients with IgA nephropathy and 3 healthy subjects after transfection with molecules of "inhibitor" (A) and "mimic" (B) mi-RNA respectively. Each expression value was normalized with the expression value of the housekeeping gene β-actin.

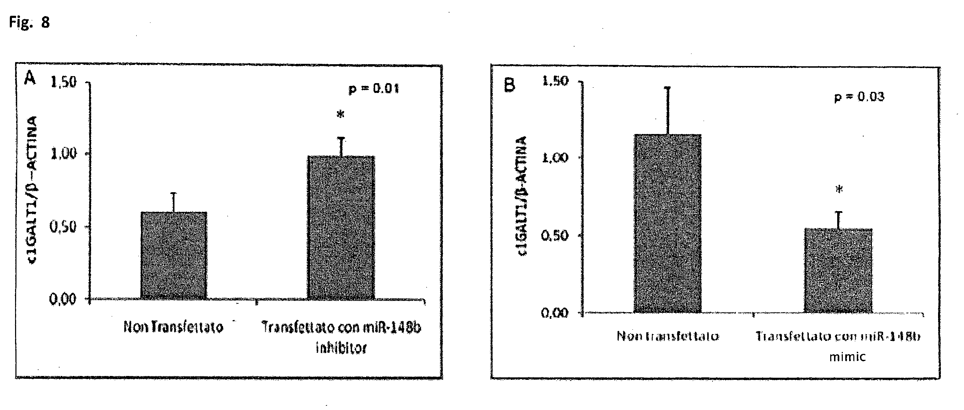

As shown in figure 8A, the expression of the gene c1GALT1 in the lymphomonocytes isolated from patients with IgA nephropathy after transfection with molecules of inhibitor miR-148b increased considerably by 3.5 times, demonstrating the actual involvement of the miR-148b in the expression of the gene c1GALT1.

For a further confirmation, the lymphomonocytes isolated from healthy subjects were transfected with molecules of "mimic" miR-148b, molecules that increase the levels of endogenous mi-RNAs. As shown in figure 8B, the levels of the gene c1GALT1 are significantly reduced after the transfection.

Fig. 8 shows the levels of the core protein 1,β-1,3-Galactosyltransferase (c1GALT1), evaluated by means of Western Blot, in the peripheral lymphomonocytes isolated from 4 patients with IgA nephropathy and 3 healthy subjects after transfection with molecules of "inhibitor" mi-RNA (antisense mi-RNA) (A) and "mimic" mi-RNA (B) respectively. Each expression value of the protein c1GALT1 with the signal corresponding to the protein β-actin.

In agreement with the expression values of the mRNA shown in figure 8 (A-B), the miR-148b actually regulates the synthesis of c1GALT1 and consequently the glycosylation of the IgA1.

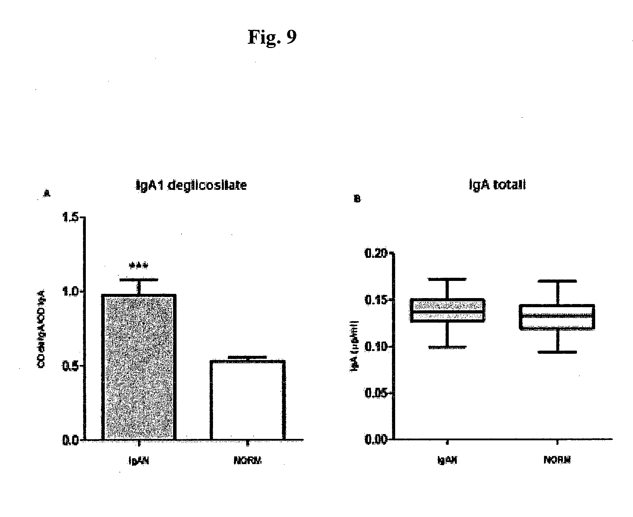

Fig. 9 shows the levels of deglycosylated IgA1 in the serum of ill subjects and in the serum of the controls, showing a significant increase in the levels of IgAl deglycosylate in the patients with IgA nephropathy with respect to the healthy subjects, although the levels of total IgA were similar in the two groups.

In particular, panel A shows the serum levels of deglycosylated IgA1(deIgA) in the patients with IgA nephropathy (IgAN) and healthy subjects (NORM). Each absorbance value (OD) of the deIgA was normalized to the absorbance values of the total IgA. The levels of deglycosylated IgAl are significantly higher in the patients with IgA nephropathy than in the healthy subjects (*** p< 0.001).

Panel B shows the serum levels of total IgA in the patients with IgA nephropathy compared to the healthy subjects; the levels of IgA in the subjects belonging to the two groups are similar. The results are shown as means of the concentration values of IgA of each sample ± SEM.

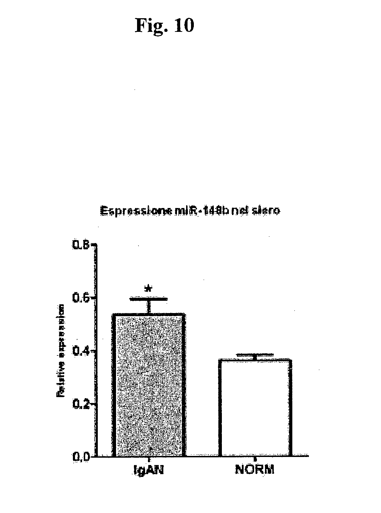

Fig. 10 shows the expression values of the miR-148b, determined by means of Real-Time PCR, starting from serum obtained from an independent group of 10 patients with IgA nephropathy (IgAN) and 10 healthy subjects (NORM). Each expression value was normalized to the expression value of the miR-27a (a miRNA also expressed in the IgAN and NORM samples).

The results are shown as means of the expression values of each sample ± SEM. As shown in the figure, the expression of the miRNA is significantly higher in the IgAN than in the healthy subjects (* p< 0.05).

## Claims

1. Use of a mi-RNA which has a sequence chosen from the sequences SEQ. ID No.: 1-6 for the diagnosis of IgA nephropathy, in a biological sample chosen from peripheral lymphomonocytes, blood and plasma.

2. Use as claimed in claim 1, **characterized in that** said mi-RNA is miR-148b having the sequence SEQ. ID No 1.

3. Method for the diagnosis of IgA nephropathy, which comprises evaluating the expression of at least one mi-RNA chosen from the sequences SEQ. ID No.: 1-6 in a biological sample chosen from peripheral lymphomonocytes, blood and plasma of a subject, in which an increase in the expression of said mi-RNA indicates the presence of IgA nephropathy or a predisposition to develop IgA nephropathy.

4. Method as claimed in claim 3, **characterized in that** said at least one mi-RNA is miR-148b having the sequence SEQ. ID No. 1.

5. Method as claimed in claim 4, **characterized in that** it comprises evaluating the expression of miR-148b having the sequence SEQ. ID No. 1 and at least one further mi-RNA chosen from the sequences SEQ. ID No. 2-6.

6. Use of a diagnostic kit comprising at least one antisense mi-RNA which has a sequence chosen from the sequences SEQ. ID No.: 7-18 for the diagnosis of IgA nephropathy.

7. Use as claimed in claim 6, **characterized in that** said at least one antisense mi-RNA is miR-148b-antisense chosen from SEQ. ID No 7 and SEQ. ID No 8.

8. Use as claimed in claim 7, **characterized in that** it further comprises at least one other antisense mi-RNA chosen from the sequences SEQ. ID No. 9-18.

9. Antisense mi-RNA for use in the treatment and/or prevention of IgA nephropathy, **characterized in that** it is antisense miR-148b chosen from SEQ. ID No 7 and SEQ. ID No 8.

## Patentansprüche

1. Verwendung einer mi-RNA mit einer Sequenz, die aus den Sequenzen SEQ.ID No.: 1-6 ausgewählt ist, zur Diagnose von IgA-Nephropathie in einer biologischen Probe, die aus peripheren Lymphomonozyten, Blut und Plasma ausgewählt ist.

2. Verwendung gemäß Anspruch 1, **dadurch gekennzeichnet, dass** die mi-RNA miR-148b mit der Sequenz SEQ.ID No. 1 ist.

3. Verfahren zur Diagnose von IgA-Nephropathie, umfassend die Evaluierung der Expression von mindestens einer mi-RNA, die aus den Sequenzen SEQ.ID No.: 1-6 ausgewählt ist, in einer biologischen Probe, die aus peripheren Lymphomonozyten, Blut und Plasma ausgewählt ist, eines Patienten, in dem eine Erhöhung der Expression der mi-RNA die Anwesenheit von IgA-Nephropathie oder eine Prädisposition, IgA-Nephropathie zu entwickeln, anzeigt.

4. Verfahren gemäß Anspruch 3, **dadurch gekennzeichnet, dass** die mindestens eine mi-RNA miR-148b mit der Sequenz SEQ.ID No. 1 ist.

5. Verfahren gemäß Anspruch 4, **dadurch gekennzeichnet, dass** dieses die Evaluierung der Expression von miR-148b mit der Sequenz SEQ.ID No. 1 und mindestens einer weiteren mi-RNA, die aus den Sequenzen SEQ.ID No.: 2-6, ausgewählt ist, umfasst.

6. Verwendung eines Diagnosekits, umfassend mindestens eine Antisense-mi-RNA mit einer Sequenz, die aus den Sequenzen SEQ.ID No.: 7-18 ausgewählt ist, zur Diagnose von IgA-Nephropathie.

7. Verwendung gemäß Anspruch 6, **dadurch gekennzeichnet, dass** die mindestens eine Antisense-mi-RNA Antisense-miR-148b ist, welche aus SEQ.ID No. 7 und SEQ.ID No. 8 ausgewählt ist.

8. Verwendung gemäß Anspruch 7, **dadurch gekennzeichnet, dass** diese außerdem mindestens eine Antisense-mi-RNA umfasst, die aus den Sequenzen SEQ.ID No.: 9-18 ausgewählt ist.

9. Antisense-mi-RNA zur Verwendung in der Behandlung und/oder der Vorbeugung von IgA-Nephropathie, **dadurch gekennzeichnet, dass** diese Antisense-miR-148b ist, welche aus SEQ.ID No. 7 und SEQ.ID No. 8 ausgewählt ist.

## Revendications

1. Utilisation d'un mi-ARN qui a une séquence choisie parmi les séquences SEQ ID NO : 1 à 6 pour le diagnostic d'une néphropathie à IgA, dans un échantillon biologique choisi parmi les lymphomonocytes périphériques, le sang et le plasma.

2. Utilisation selon la revendication 1, **caractérisée en ce que** ledit mi-ARN est le miR-148b ayant la séquence SEQ ID NO : 1.

3. Procédé de diagnostic d'une néphropathie à IgA, qui comprend l'évaluation de l'expression d'au moins un mi-ARN choisi parmi les séquences SEQ ID NO : 1 à 6 dans un échantillon biologique choisi parmi les lymphomonocytes, le sang et le plasma d'un sujet, dans lequel une augmentation de l'expression dudit mi-ARN indique la présence d'une néphropathie à IgA ou une prédisposition à développer une néphropathie à IgA.

4. Procédé selon la revendication 3, **caractérisé en ce que** ledit au moins un mi-ARN est le miR-148b ayant la séquence SEQ ID NO : 1.

5. Procédé selon la revendication 4, **caractérisé en ce qu'**il comprend l'évaluation de l'expression de miR-148b ayant la séquence SEQ ID NO : 1 et d'au moins un autre mi-ARN choisi parmi les séquences SEQ ID NO : 2 à 6.

6. Utilisation d'un kit de diagnostic comprenant au moins un mi-ARN antisens qui a une séquence choisie parmi les séquences SEQ ID NO : 7 à 18 pour le diagnostic d'une néphropathie à IgA.

7. Utilisation selon la revendication 6, **caractérisée en ce que** ledit au moins un mi-ARN antisens est le miR-148b antisens choisi parmi SEQ ID NO : 7 et SEQ ID NO : 8.

8. Utilisation selon la revendication 7, **caractérisée en ce qu'**elle comprend en outre au moins un autre mi-ARN antisens choisi parmi les séquences SEQ ID NO : 9 à 18.

9. mi-ARN antisens pour une utilisation dans le traitement et/ou la prévention d'une néphropathie à IgA, **caractérisé en ce qu'**il s'agit du miR-148b antisens choisi parmi SEQ ID NO : 7 et SEQ ID NO : 8.
